# EUROPÄISCHE PATENTSCHRIFT

(11) **EP 1 706 411 B1**
(45) Veröffentlichungstag und Bekanntmachung des Hinweises auf die Patenterteilung: **24.02.2010**
(21) Anmeldenummer: 04804410.1
(22) Anmeldetag: 30.12.2004
(51) Int. Cl.: C07D 487/22

(54) **VERFAHREN ZUR HERSTELLUNG VON PHTHALOCYANINEN**
METHOD FOR PREPARING PHTHALOCYANINES
METHODE DE PREPARATION DE PHTALOCYANINES

(30) Priorität: 08.01.2004 DE 102004001457
(43) Veröffentlichungstag der Anmeldung: 04.10.2006
(73) Patentinhaber: BASF SE, 67056 Ludwigshafen (DE)
(72) Erfinder: GESSNER, Thomas, 69120 Heidelberg (DE); EBERT, Sophia, 68165 Mannheim (DE)
(86) Internationale Anmeldenummer: PCT/EP2004/014825
(87) Internationale Veröffentlichungsnummer: WO 2005/066179

(56) Entgegenhaltungen:
- EP-A- 0 663 427
- US-A- 2 413 191
- US-A- 3 509 146
- P. J. BRACH ET AL: "Improved Synthesis of Metal-free Phthalocyanines" JOURNAL OF HETEROCYCLIC CHEMISTRY, Bd. 7, 1970, Seiten 1403-1405, XP002325080 in der Anmeldung erwähnt

## Beschreibung

Die vorliegende Erfindung betrifft ein Verfahren zur Herstellung von metallfreien Phthalocyaninen der Formel I 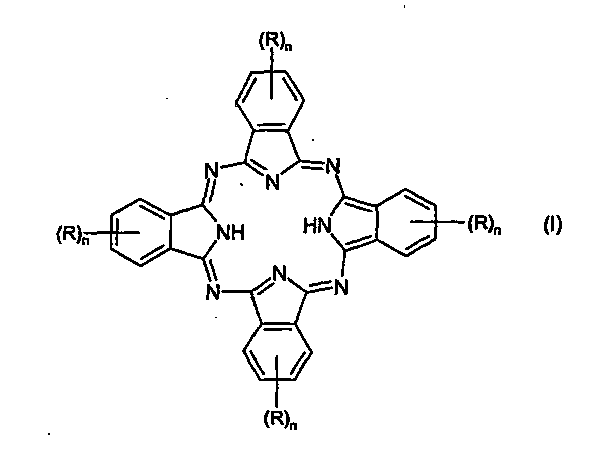 durch Umsetzung eines ortho-Phthalodinitrils der Formel Ia 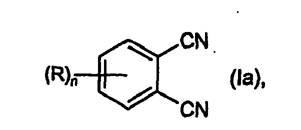 in einem inerten Lösungsmittel mit einer Siedetemperatur von wenigstens 120°C (unter Normaldruck) in Gegenwart von Ammoniak,
wobei in Formel I bzw. la die Variable n Werte von 1, 2, 3 oder 4 annehmen kann und die Reste R einen gegebenenfalls mit ein oder zwei C₁-C₈-Alkylgruppen substituierten fünf- oder sechsgliedrigen gesättigten stickstoffhaltigen heterocyclischen Ring bezeichnen, der über ein Ringstickstoffatom an den Benzolring gebunden ist und der noch ein oder zwei weitere Stickstoffatome oder ein weiteres Sauerstoff- oder Schwefelatom enthalten kann,
welches dadurch gekennzeichnet ist, dass die Umsetzung in Gegenwart eines Alkalimetallhydroxids oder Alkalimetallcarbonats durchgeführt wird.

Die Herstellung von metallfreien Phthalocyanin erfolgt in der Regel in einem hochsiedenden Lösungsmittel ausgehend von Isoindolenindiiminen, wie etwa in der Schrift US 3,509,146 beschrieben, oder ausgehend von o-Phthalodinitril oder Isoindolenindiiminen in Gegenwart einer Base, beispielsweise Ammoniak, wie etwa in P. J. Brach, S. J. Grammatica, O. A. Ossanna und L. Weinberger, J. Heterocyclic Chem. 7 (1970), 1403 - 1405 ausgeführt.

Die Herstellung von metallfreien Phthalocyaninen der Formel I in Anlehnung an die Herstellverfahren, wie sie in den zuvor genannten Schriften dargelegt sind, führt jedoch zu unbefriedigenden Ausbeuten. So konnte beispielsweise das 1(4),8(11),15(18), 22(25)-Tetra(3-methylpiperidino)-phthalocyanin ausgehend von 3-(3-Methylpiperidino)-phthalodinitril nach der Vorschrift von P. J. Brach et al. zwar in hoher Reinheit, aber nur in einer geringen Ausbeute von 37 % erhalten werden.

Die Aufgabe der vorliegenden Erfindung bestand somit darin, ein Verfahren bereitzustellen, mit welchem metallfreie Phthalocyanine der Formel I in hoher Reinheit und Ausbeute hergestellt werden können. Diese Aufgabe wurde durch das eingangs beschriebene Verfahren gelöst.

Bei den Resten R der Formeln I und Ia handelt es sich um gegebenenfalls mit ein oder zwei C₁-C₈-Alkylgruppen substituierte fünf- oder sechsgliedrige gesättigte stickstoffhaltige heterocyclische Ringe, welche über ein geeignetes Ringstickstoffatom an den Benzolring gebunden sind und noch ein oder zwei weitere Stickstoffatome oder ein weiteres Sauerstoff- oder Schwefelatom enthalten können.

Bevorzugt handelt es sich bei den Resten R um gegebenenfalls mit ein oder zwei C₁-C₄-Alkylgruppen substituierte sechsgliedrige gesättigte stickstoffhaltige heterocyclische Ringe, welche über ein Ringstickstoffatom an den Benzolring gebunden sind und noch ein weiteres Stickstoffatom enthalten können.

Beispiele solcher heterocyclischen Ringe sind Pyrrolidin-1-yl, 2- oder 3-Methylpyrrolidin-1-yl, 2,4-Dimethyl-3-ethylpyrrolidin-1-yl, Pyrazolidin-1-yl, 2-, 3-, 4- oder 5-Methylpyrazolidin-1-yl, Imidazolidin-1-yl, 2-, 3-, 4- oder 5-Methylimidazolidin-1-yl, Oxazolidin-3-yl, 2-, 4- oder 5-Methyloxazolidin-3-yl, Isoxazolidin-2-yl, 3-, 4- oder 5-Methylisoxazolidin-2-yl, Piperidin-1-yl, (C₁-C₄-Alkyl)piperidin-1-yl, wie 2-,3-,4-Methyl-oder-Ethylpiperidin-1-yl, 2,6-Dimethylpiperidin-1-yl, Piperazin-1-yl, 4-(C₁-C₄-Alkyl)piperazin-1-yl, wie 4-Methyl- oder 4-Ethylpiperazin-1-yl, Morpholin-4-yl, Thiomorpholin-4-yl oder Thiomorpholin-4-yl-S,S-dioxid ab.

Besonders bevorzugt handelt es sich bei R um mit ein oder zwei C₁-C₄-Alkylgruppen substituierte Piperidin-1-yl- oder Piperazin-1-yl-Reste.

C₁-C₈- bzw. C₁-C₄-Alkylgruppen als mögliche Substituenten der heterocyclischen Ringe sind beispielsweise Methyl, Ethyl, Propyl, Isopropyl, Butyl, Isobutyl, sec-Butyl, tert-Butyl, Pentyl, Isopentyl, Neopentyl, tert-Pentyl, Hexyl, 2-Methylpentyl, Heptyl, Hept-3-yl, Octyl, 2-Ethylhexyl und Isooctyl.

Grundsätzlich ist verfahrensgemäß die Verwendung von Mischungen von verschiedenen Verbindungen der Formel Ia möglich, welche sich jeweils in den Werten von n und/oder der chemischen Natur ihrer Reste R und/oder deren relativen Positionen zu den Nitrilgruppen des Phthalodinitrils voneinander unterscheiden.

Vorzugsweise handelt es sich bei der Verbindung der Formel Ia jedoch um eine Reinverbindung mit gegebenem Wert der Variablen n, wobei für n gleich 2, 3 oder 4 die Reste R vorzugsweise identisch sind.

Besonders bevorzugt nimmt in Formel I bzw. Ia die Variable n den Wert 1 an.

Erwähnt sei in diesem Zusammenhang noch, dass nicht nur für chemisch verschiedene Reste R sondern auch im letzteren, bevorzugten Fall identischer Reste R die resultierende Verbindung der Formel I aus einem Gemisch von Stellungsisomeren bestehen kann. Dies wird in den nachfolgenden Beispielen (vgl. "B) Umsetzung in n-Butylglykol") exemplarisch ausgeführt.

Als inerte Lösungsmittel kommen alle dem Fachmann aus dem Stand der Technik für die Herstellung von metallfreien Phthalocyaninen bekannten Lösungsmittel in Frage sofern sie eine Siedetemperatur von wenigstens 120°C (unter Normaldruck) besitzen.

Bevorzugt setzt man im erfindungsgemäßen Verfahren Lösungsmittel ausgewählt aus der Gruppe bestehend aus Ethylenglykol, Diethylenglykol, Propylenglykol, 1,2-Butandiol, 1,3-Butandiol, 1,4-Butandiol, 2,3-Butandiol, den Mono- und Di(C₁-C₄-alkyl)ethern der zuvor genannten Diole, 2-Di(C₁-C₄-alkyl)aminoethanol und 3-Di(C₁-C₄-alkyl)aminopropanol. Als C₁-C₄-Alkylreste der Mono- und Di(C₁-C₄-alkyl)ether der genannten Diole sowie der 2-Di(C₁-C₄-alkyl)aminoethanole und 3-Di(C₁-C₄-alkyl)aminopropanole kommen in Betracht Methyl, Ethyl, n-Propyl, i-Propyl, n-Butyl, i-Butyl, sec.-Butyl, tert.-Butyl. Bei den Alkylresten handelt es sich im Falle der Mono- und Diether in der Regel um Methyl oder n-Butyl, im Falle der Aminoalkohole um Methyl. Vorteilhaft finden die Monoether der genannten Diole und 3-Di(C₁-C₄-alkyl)aminopro-panole Verwendung.

Bei der Auswahl eines spezifischen Lösungsmittels aus der zuvor genannten Gruppe ist natürlich zusätzlich die Maßgabe zu beachten, dass es eine Siedetemperatur von wenigstens 120°C besitzen muss.

Besonders bevorzugt sind n-Butylglykol und 3-Dimethylamino-propanol.

Als Alkalimetallhydroxid oder Alkalimetallcarbonat finden bevorzugt Natriumhydroxid, Kaliumhydroxid, Natriumcarbonat und Kaliumcarbonat, besonders bevorzugt Natriumhydroxid und Kaliumcarbonat Verwendund.

Verfahrensgemäß wird üblicherweise eine der genannten Basen zugesetzt, es können jedoch auch Mischungen aus zwei oder mehreren Basen Verwendung finden.

Der Anteil der Base oder Basenmischung beträgt üblicherweise 0,5 bis 10 mol-%, vorzugsweise 1 bis 6 mol-%, bezogen auf die Molzahl der Verbindung der Formel Ia.

Die erfindungsgemäße Umsetzung wird üblicherweise in gängigen Reaktoren mit entsprechenden Rührvorrichtungen sowie gegebenenfalls die Durchmischung verbessernden Einbauten, wie etwa Strömungsbrechem, durchgeführt.

Der Ammoniak wird üblicherweise mit konstantem Volumenstrom am Boden des Reaktors in die Reaktionsmischung eingeleitet. Die Eichung der pro Zeiteinheit dosierten Ammoniakmenge kann hierbei mittels üblicher Methoden, z.B. durch Auffangen in verdünnter Essigsäure und anschließende Titration, vorgenommen werden.

Die Menge an Ammoniak beträgt vorzugsweise mindestens zwei Moläquivalente bezogen auf die Molzahl an Verbindung der Formel Ia, da davon ausgegangen wird, dass der Ammoniak entsprechend der nachfolgenden chemischen Gleichung katalytisch wirkt:

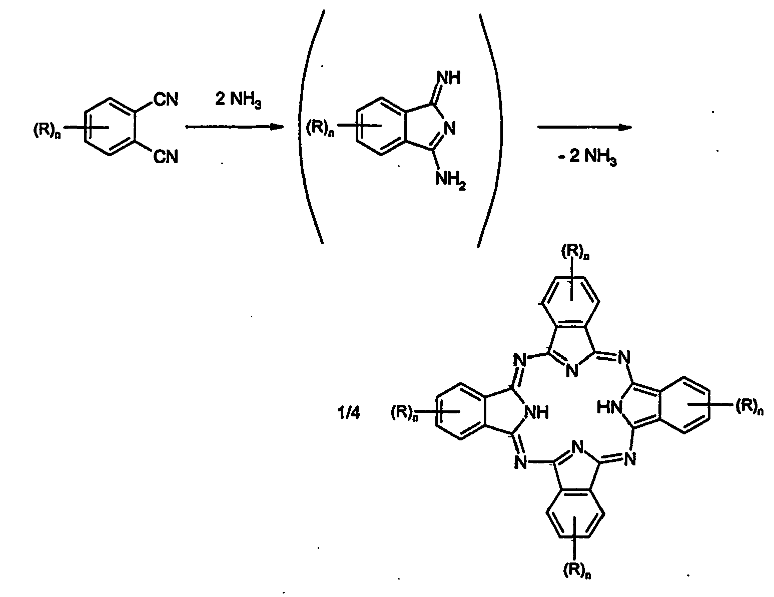

Die Dauer der Ammoniakeinleitung liegt üblicherweise bei mehreren Stunden. Als Orientierungshilfe können hierbei die im Labormaßstab seitens der Anmelderin durchgeführten Versuche dienen. Beispielsweise wurde sowohl in einem 500-ml-Rundkolben mit Blattrührer als auch in einem 2-1-Reaktor mit Scheibenrührer und Strömungsbrechern die Mindestmenge von zwei Moläquivalenten Ammoniak nach einer Gesamteinleitungsdauer von 9 Stunden (2 Stunden während der Aufheizphase und 7 Stunden bei Endtemperatur) erreicht, wobei die Eingasung getaucht am Boden des Kolbens bzw. Reaktors erfolgte.

Die Reaktionstemperatur liegt üblicherweise zwischen 140 und 170°C, doch lässt sich die für ein spezifisches, inertes Lösungsmittel am besten geeignete Reaktionstemperatur vom Fachmann in einfacher Weise durch routinemäßige Vorversuche ermitteln. Beispielsweise wurden in Versuchen der Anmelderin mit den Lösungsmitteln 3-Dimethylaminopropanol und n-Butylglykol die größten Ausbeuten bei Reaktionstemperaturen von etwa 150 °C und 160°C respektive ermittelt.

Das Verhältnis von Verbindung der Formel la (Molzahl) zu inertem Lösungsmittel (Volumen) liegt üblicherweise bei etwa zwei Mol zu einem Liter, kann jedoch im Einzelfall sowohl über- als auch unterschritten werden.

### Beispiele:

### Herstellung von 1(4),8(11),15(18),22(25)-Tetra-(3-methylpiperidino)-phthalocyanin:

### A) Umsetzung in 3-Dimethylaminopropanol:

225.3 g (1.00 mol) 3-(3-Methylpiperidino)-phthalodinitril wurden in 500 ml 3-Dimethylaminopropanol bei Raumtemperatur unter Rühren (150 U/min) in einem 2-1-Planschliffkessel eingetragen. Anschließend gab man 4.85 g (0.035 mol; 3.5 mol-%) Kaliumcarbonat zu. Insgesamt 34.1 g (2.00 mol) Ammoniak wurden in die Reaktionsmischung über ein Tauchrohr 9 Stunden (2h während der Aufheiz- und 7h während der Reaktionsphase) lang mit einem Volumenstrom von ca. 83 ml/min eingegast, wobei die Reaktionsmischung auf eine Endtemperatur von 150°C erhitzt und 15 Stunden bei dieser Temperatur gehalten wurde. Danach kühlte man die schwarze Reaktionslösung auf 50°C ab und versetzte innerhalb von 2 Stunden unter Rühren mit 1000 ml Methanol, um den beim Abkühlen entstandenen Feststoff vollständig auszufällen. Die Suspension wurde eine Stunde lang bei 50°C nachgerührt, dann auf Raumtemperatur gekühlt und über eine Nutsche filtriert. Der Filterkuchen wurde erst mit 800 ml Methanol, dann mit 1000 ml Wasser gewaschen und schließlich trockengesaugt.

Nach dem Trocknen bei 60°C im Vakuum wurde ein schwarzes Pulver in einer Ausbeute von 170.4 g (70 % d.Th. bezogen auf die Reinsubstanz) erhalten.

Eine dreimal aus 3-Dimethylaminopropanol und einmal aus n-Butylglykol umkristallisierte Probe war praktisch analysenrein. Die Elementaranalyse ergab:

| | | | |
|---|---|---|---|
| C₅₆H₆₂N₁₂ | ber. C 74.47% | H 6.92% | N 18.61% |
| (903.2 g/mol) | gef. C 74.8% | H 6.9% | N 18.2% |

### B) Umsetzung in n-Butylglykol:

Wurde anstelle des 3-Dimethylaminopropanols für die Umsetzung als inertes Lösungsmittel n-Butylglykol verwendet und als Endtemperatur 160°C eingestellt (bei ansonsten unveränderten übrigen Parametern gegenüber der Versuchsführung gemäß A)), so erhielt man vergleichbare Ausbeuten und Reinheiten an dem gewünschten Produkt, welches (wie auch gemäß A) als Mischung verschiedener Stellungsisomerer der nachfolgenden Strukturen vorlag:

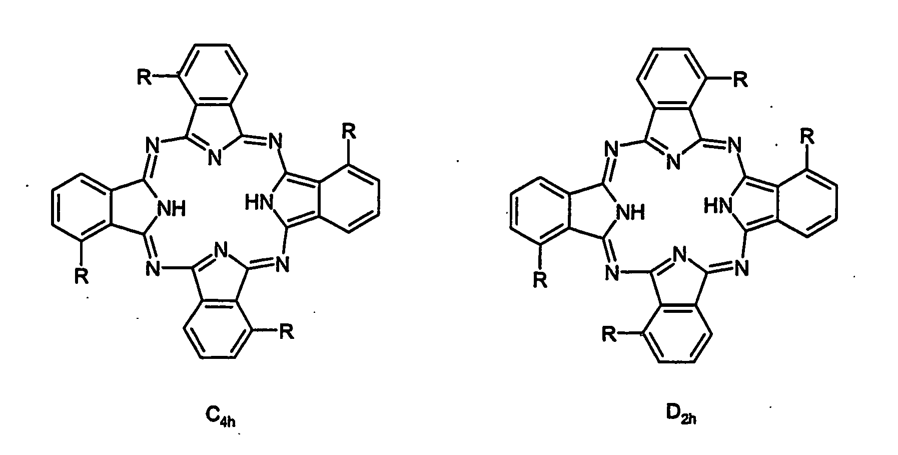

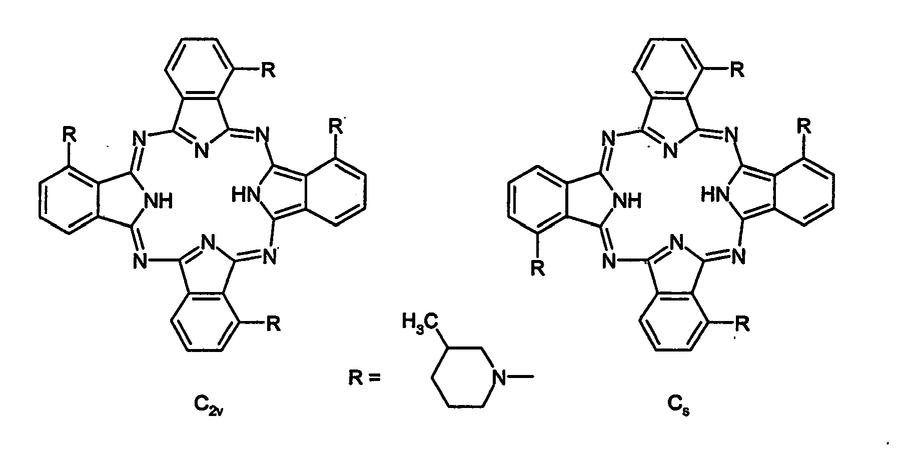

## Patentansprüche

1. Verfahren zur Herstellung von metallfreien Phthalocyaninen der Formel I 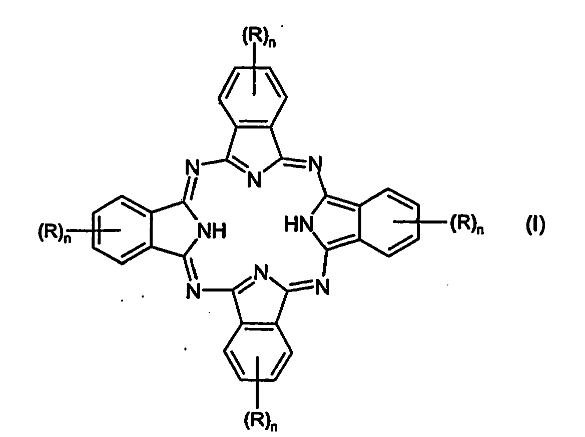 durch Umsetzung eines ortho-Phthalodinitrils der Formel la 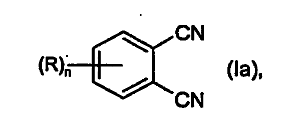 in einem inerten Lösungsmittel mit einer Siedetemperatur von wenigstens 120°C unter Normaldruck in Gegenwart von Ammoniak,
wobei in Formel I bzw. la die Variable n Werte von 1, 2, 3 oder 4 annehmen kann und die Reste R einen gegebenenfalls mit ein oder zwei C₁-C₈-Alkylgruppen substituierten fünf- oder sechsgliedrigen gesättigten stickstoffhaltigen heterocyclischen Ring bezeichnen, der Ober ein Ringstickstoffatom an den Benzolring gebunden ist und der noch ein oder zwei weitere Stickstoffatome oder ein weiteres Sauerstoff- oder Schwefelatom enthalten kann,
**dadurch gekennzeichnet, dass** die Umsetzung in Gegenwart eines Alkalimetallhydroxids oder Alkalimetallcarbonats durchgeführt wird.

2. Verfahren nach Anspruch 1, **dadurch gekennzeichnet, dass** das inerte Lösungsmittel ausgewählt ist aus der Gruppe bestehend aus Ethylenglykol, Diethylenglykol, Propylenglykol, 1,2-Butandiol, 1,3-Butandiol, 1,4-Butandiol, 2,3-Butandiol, den Mono- und Di(C₁-C₄-alkyl)ethern der zuvor genannten Diole, 2-Di(C₁-C₄-alkyl)aminoethanol und 3-Di(C₁-C₄-alkyl)aminopropanol.

3. Verfahren nach Anspruch 1 oder 2, **dadurch gekennzeichnet, dass** als inertes Lösungsmittel 3-Dimethylaminopropanol oder n-Butylglykol verwendet werden.

4. Verfahren nach einem oder mehreren der vorherigen Ansprüche, **dadurch gekennzeichnet, dass** als Alkalimetallhydroxid oder Alkalimetallcarbonat Natriumhydroxid, Kaliumhydroxid, Natriumcarbonat oder Kaliumcarbonat verwendet werden.

5. Verfahren nach einem oder mehreren der vorherigen Ansprüche, **dadurch gekennzeichnet, dass** n in den Formeln I und Ia den Wert 1 annimmt.

6. Verfahren nach einem oder mehreren der vorherigen Ansprüche, **dadurch gekennzeichnet, dass** die Reste R einen mit ein oder zwei C₁-C₄-Alkylgruppen substituierten sechsgliedrigen gesättigten stickstoffhaltigen heterocyclischen Ring bezeichnen, der über ein Ringstickstoffatom an den Benzolring gebunden ist und der noch ein weiteres Stickstoffatom enthalten kann.

7. Verfahren nach einem oder mehreren der vorherigen Ansprüche, **dadurch gekennzeichnet, dass** die Reste R einen mit ein oder zwei C₁-C₄-Alkylgruppen substituierten Piperidin- oder Piperazinring bezeichnen, der über das Ringstickstoffatom bzw. eines der beiden Ringstickstoffatome an den Benzolring gebunden ist.

## Claims

1. A process for the preparation of metal-free phthalocyanines of the formula I 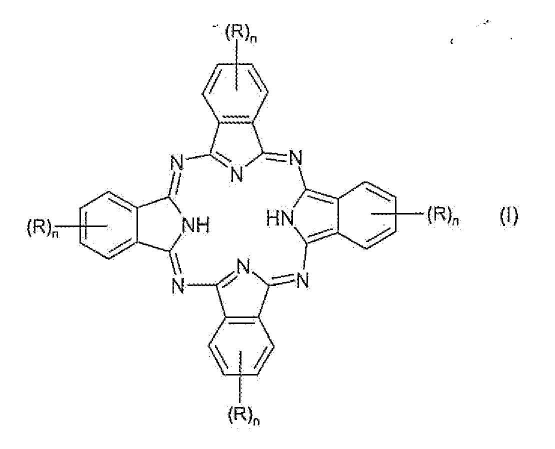 by conversion of an ortho-phthalodinitrile of the formula Ia 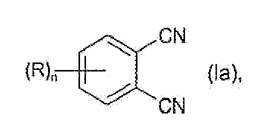 in an inert solvent with a boiling point of at least 120°C at standard pressure in the presence of ammonia,
in which, in formula I or Ia, the variable n can adopt values of 1, 2, 3 or 4 and the R radicals denote a five- or six-membered saturated nitrogen-comprising heterocyclic ring optionally substituted by one or two C₁-C₈-alkyl groups which is bonded via a ring nitrogen atom to the benzene ring and which can still comprise one or two additional nitrogen atoms or an additional oxygen or sulfur atom,
which comprises carrying out the conversion in the presence of an alkali metal hydroxide or alkali metal carbonate.

2. The process according to claim 1, wherein the inert solvent is chosen from the group consisting of ethylene glycol, diethylene glycol, propylene glycol, 1,2-butanediol, 1,3-butanediol,1,4-butanediol, 2,3-butanediol, the mono- and di (C₁-C₄-alkyl) ethers of the abovementioned diols, 2- [di (C₁-C₄-alkyl) amino] ethanol and 3 - [di (C₁-C₄-alkyl) amino] propanol.

3. The process according to claim 1 or 2, wherein 3-dimethylaminopropanol or n-butyl glycol is used as inert solvent.

4. The process according to one or more of the preceding claims, wherein sodium hydroxide, potassium hydroxide, sodium carbonate or potassium carbonate is used as alkali metal hydroxide or alkali metal carbonate.

5. The process according to one or more of the preceding claims, wherein n in the formulae I and Ia adopts the value 1.

6. The process according to one or more of the preceding claims, wherein the R radicals denote a six-membered saturated nitrogen-comprising heterocyclic ring substituted by one or two C₁-C₄-alkyl groups which is bonded via a ring nitrogen atom to the benzene ring and which can still comprise an additional nitrogen atom.

7. The process according to one or more of the preceding claims, wherein the R radicals denote a piperidine or piperazine ring substituted by one or two C₁-C₄-alkyl groups which is bonded via the ring nitrogen atom or one of the two ring nitrogen atoms to the benzene ring.

## Revendications

1. Procédé pour la préparation de phtalocyanines exemptes de métal de formule I 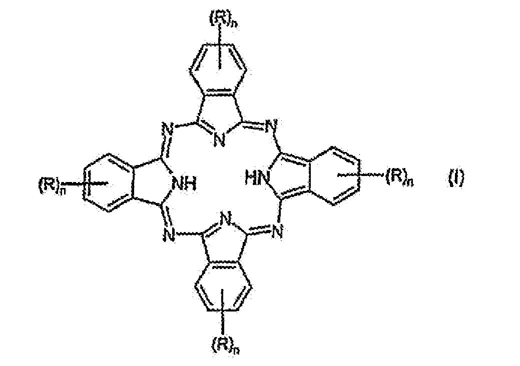 par transformation d'un ortho-phtalodinitrile de formule Ia 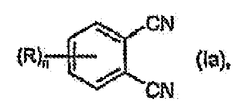 dans un solvant inerte présentant une température d'ébullition d'au moins 120°C à pression normale en présence d'ammoniac,
où, dans la formule I ou, selon le cas, Ia la variable n peut présenter les valeurs 1, 2, 3 ou 4 et les radicaux R désignent un cycle hétérocyclique' azoté, saturé, à cinq ou six chaînons, le cas échéant substitué par un ou deux groupes C₁-C₈-alkyle, qui est lié au cycle benzène via un atome d'azote du cycle et qui peut encore contenir un ou deux autres atomes d'azote ou un autre atome d'oxygène ou de soufre,
**caractérisé en ce que** 1a transformation est réalisés en présence d'un hydroxyde de métal alcalin ou d'un carbonate de métal alcalin.

2. Procédé selon la revendication 1, **caractérisé en ce que** le solvant inerte est choisi dans le groupe constitué par l'éthylèneglycol, le diéthylèneglycol, le propylèneglycol, le 1,2-butanediol, le 1,3-butanediol, le 1,4-butanediol, le 2,3-butanediol, les mono(C₁-C₄-alkyl)éthers et les di (C₁-C₄-alkyl) éthers des diols susmentionnés, le 2-di(C₁-C₄-alkyl)aminoéthanol et le 3-di (C₁-C₄-alkyl) aminopropanol.

3. Procédé selon la revendication 1 ou 2,
**caractérisé en ce qu'**on utilise comme solvant inerte le 3-diméthylaminopropanol ou le n-butylglycol.

4. Procédé selon l'une ou plusieurs des revendications précédentes, **caractérisé en ce qu'**on utilise comme hydroxyde de métal alcalin ou carbonate de métal alcalin l'hydroxyde de sodium, l'hydroxyde de potassium, le carbonate de sodium ou le carbonate de potassium.

5. Procédé selon l'une ou plusieurs des revendications précédentes, **caractérisé en ce que** n dans les formules I et Ia présente la valeur 1.

6. Procédé selon l'une on plusieurs des revendications précédentes, **caractérisé en ce que** les radicaux R désignent un cycle hétérocyclique azoté, saturé, à six chaînons, substitué par un ou deux groupes C₁-C₄-alkyle, qui est lié au cycle benzène via un atome d'azote du cycle et qui peut encore contenir un autre atome d'azote.

7. Procédé selon l'une ou plusieurs des revendications précédentes, **caractérisé en ce que** les radicaux R désignent un cycle pipéridine ou pipérazine substitué par un ou deux groupes C₁-C₄-alkyle, qui est lié au cycle benzène via l'atome d'azote du cycle ou, selon le cas, un des deux atomes d'azote du cycle.
